# EUROPEAN PATENT SPECIFICATION

(11) **EP 3 420 897 B1**
(45) Date of publication and mention of the grant of the patent: **16.03.2022**
(21) Application number: 16891658.3
(22) Date of filing: 16.12.2016
(51) Int. Cl.: A61M 25/00

(54) **ELECTRODE CATHETER**
ELEKTRODENKATHETER
CATHÉTER À ÉLECTRODES

(30) Priority: 25.02.2016 JP 2016034868; 25.05.2016 JP 2016104152
(43) Date of publication of application: 02.01.2019
(73) Proprietor: Japan Lifeline Co., Ltd., Tokyo 140-0002 (JP)
(72) Inventor: MASUDA, Takuya, Tokyo 140-0002 (JP); SASAKI, Takuya, Tokyo 140-0002 (JP)
(74) Representative: Grünecker Patent- und Rechtsanwälte PartG mbB
(86) International application number: PCT/JP2016/087523
(87) International publication number: WO 2017/145503

(56) References cited:
- WO-A1-2007/114418
- JP-A- H09 140 660
- JP-A- 2009 254 423
- JP-A- 2009 268 696
- JP-A- 2012 034 852
- US-A- 5 626 136
- US-A1- 2010 331 821

## Description

### Technical Field

The present invention relates to an electrode catheter and, more specifically, relates to an electrode catheter that comprises a catheter shaft constituted by a braided tube.

### Background Art

In a catheter shaft that constitutes an electrode catheter such as an EP catheter or the like, a plurality of ring-shaped electrodes are attached to the outer circumferential surface of the distal end part thereof.

Leads that are each coupled to the plurality of ring-shaped electrodes enter a lumen of the catheter shaft through side holes formed in the tube wall of the shaft in correspondence to the attachment positions of the ring-shaped electrodes and extend in said lumen, and the proximal ends of the leads are each coupled to terminals disposed in the inner part of a connector that is coupled to the proximal end side of the catheter shaft.

Hitherto, an electrode catheter in which the proximal end part of a catheter shaft is constituted by a resin tube (braided tube) reinforced by a braid made of a metal such as stainless steel is known (refer to, for example, PTL 1 presented below).

It is possible to improve the torque transmission of the electrode catheter by a certain degree by constituting the proximal end part of the catheter shaft by the braided tube.

JP 2012 034852 A discloses a catheter having an electrode which is connected with a lead through an opening in the side wall of the tube. The lead has an insulation coating. There is further disclosed a proximal braided tube part.

A further catheter in which at a tip portion different electrodes are provided is disclosed in US 5 626 136 A. These electrodes are contacted by electrode lead wires having insulation portions.

A further catheter is disclosed in US 2010/331821 A1.

### Citation List

### Patent Literature

PTL 1: Japanese Unexamined Patent Application Publication No. 2013-123508
JP 2012 034852 A
US 5 626 136 A
US 2010/331821 A1

### Summary of Invention

### Technical Problem

However, even in the electrode catheter that comprises the catheter shaft in which the proximal end part is constituted by the braided tube, the torque transmission thereof is not sufficient and is desired to be further improved.

In particular, an electrode catheter to be inserted into the inside of a body by using a guide wire is not capable of exhibiting sufficient torque transmission because a large-diameter central lumen is formed as a guide-wire lumen in a catheter shaft that constitutes the electrode catheter, and thus, the ratio of space to the shaft is high (the ratio of a resin that constitutes the shaft is low); therefore, improvement in torque transmission is an important problem in the electrode catheter that has the guide-wire lumen.

For such a problem, it is conceivable to reinforce not only the proximal end part of the catheter shaft but also the distal end part thereof with a braid made of a metal.

However, when the distal end part of the catheter shaft is constituted by such a braided tube, machining (mechanical drilling machining or drilling machining by laser or the like) to form, with respect to the tube wall of said braided tube in which the resin and the metal are complexed, a side hole through which a lead of a ring-shaped electrode passes becomes extremely difficult.

In addition, even when a side hole can be formed, metal wire materials that constitute the braid is exposed on the inner circumferential surface of said side hole, and thus, it is conceivable that, during manufacture and usage of the electrode catheter, the lead of the ring-shaped electrode may come into contact with said metal wire materials, a resin covering layer that constitutes the lead may be damaged, and the insulation of said lead may be degraded.

The present invention is made on the basis of the above circumstances.

An object of the present invention is to provide an electrode catheter that is excellent in terms of torque transmission compared with a hitherto publicly known electrode catheter in which the proximal end part of a catheter shaft is constituted by a braided tube reinforced by a braid made of a metal; that is easy in terms of machining to form, in the tube wall of the distal end part of the catheter shaft, a side hole through which a lead of a ring-shaped electrode passes; and in which a resin covering layer of the lead inserted into the formed side hole is prevented from being damaged by wire materials that constitute a braid and that are exposed on the inner circumferential surface of the side hole.

Another object of the present invention is to provide an electrode catheter that comprises a catheter shaft having a guide-wire lumen and that is excellent in terms of torque transmission.

### Solution to Problem

In particular, it is provided an electrode catheter having the features defined in claim 1. Further preferred embodiments are defined in the dependent claims.
(1) An electrode catheter of the present invention comprises a catheter shaft that has at least one lumen; at least one ring-shaped electrode that is attached to an outer circumferential surface of a distal end part of the catheter shaft; and a lead that is coupled to the ring-shaped electrode,
   in which a side hole that extends from the outer circumferential surface to the lumen is formed in a tube wall of the distal end part of the catheter shaft in correspondence to an attachment position of the ring-shaped electrode,
   in which the lead is configured by coating a metal core wire with a resin, the lead (the metal core wire) being joined at a distal end portion thereof to an inner circumferential surface of the ring-shaped electrode and thereby being coupled to said ring-shaped electrode while entering the lumen through the side hole and extending in said lumen, and
   in which the catheter shaft is constituted by a braided tube that is reinforced by a braid made of resin throughout an entire length thereof.

In the electrode catheter of the present invention, preferably, the catheter shaft has a guide-wire lumen.

It is effective to employ the constitution of the present invention in a case in which a catheter shaft has a guide-wire lumen, as is in the electrode catheter that has such a constitution, and the ratio of a resin that constitutes the shaft is low.

(3) In the electrode catheter of the present invention, preferably, the catheter shaft comprises an inside tube that has a guide-wire lumen and an outside tube that forms, in cooperation with an outer circumferential surface of the inside tube, a lumen into which the lead is inserted, the outside tube being the braided tube.

According to the electrode catheter that has such a constitution, despite the low ratio of the resin that constitutes the catheter shaft, it is possible to exhibit excellent torque transmission, as is clear from the results of the examples, which will be described later.

(4) In the electrode catheter of the present invention, preferably, the catheter shaft is a multi-lumen tube that has a plurality of lumens inside the braid.

### Advantageous Effects of Invention

According to the electrode catheter of the present invention, it is possible to exhibit more excellent torque transmission than a hitherto publicly known electrode catheter in which the proximal end part of a catheter shaft is constituted by a braided tube that is reinforced by a braid made of a metal.

In addition, it is possible to easily machine to form, in the tube wall of the distal end part of the catheter shaft, the side hole through which the lead of the ring-shaped electrode passes, and the resin covering layer of the lead inserted into the formed side hole is prevented from being damaged by the resin wire materials exposed on the inner circumferential surface of said side hole.

In addition, the electrode catheter of the present invention is excellent in terms of torque transmission although the catheter shaft that has the guide-wire lumen is comprised.

### Brief Description of Drawings

[Fig. 1] Fig. 1 is a side view illustrating an electrode catheter according to a first embodiment of the present invention.
[Fig. 2] Fig. 2 is a side view illustrating a catheter shaft that constitutes the electrode catheter illustrated in Fig. 1.
[Fig. 3A] Fig. 3A is a cross-sectional view (IIIA-IIIA sectional view) of the catheter shaft illustrated in Fig. 2.
[Fig. 3B] Fig. 3B is a cross-sectional view (IIIB-IIIB sectional view) of the catheter shaft illustrated in Fig. 2.
[Fig. 3C] Fig. 3C is a cross-sectional view (IIIC-IIIC sectional view) of the catheter shaft illustrated in Fig. 2.
[Fig. 4] Fig. 4 is a sectional view schematically illustrating a state in which a lead of a ring-shaped electrode passes through a side hole and enters a lumen of the catheter shaft in the electrode catheter illustrated in Fig. 1.
[Fig. 5] Fig. 5 is a sectional view illustrating a coupled state of an inside tube that constitutes the catheter shaft illustrated in Fig. 2 and a distal end tip.
[Fig. 6] Fig. 6 is a side view illustrating an electrode catheter according to a second embodiment of the present invention.
[Fig. 7A] Fig. 7A is a cross-sectional view (VIIA-VIIA sectional view) of a catheter shaft that constitutes the electrode catheter illustrated in Fig. 6.
[Fig. 7B] Fig. 7B is a cross-sectional view (VIIB-VIIB sectional view) of the catheter shaft that constitutes the electrode catheter illustrated in Fig. 6.
[Fig. 8] Fig. 8 is a graph showing results of evaluation tests of the torque transmission of electrode catheters according to an example and a comparative example.
[Fig. 9] Fig. 9 is a side view illustrating an electrode catheter according to example not part of the present invention.
[Fig. 10] Fig. 10 is a side view illustrating a catheter shaft that constitutes the electrode catheter illustrated in Fig. 9.
[Fig. 11A] Fig. 11A is a cross-sectional view (XIA-XIA sectional view) of the catheter shaft illustrated in Fig. 10.
[Fig. 11B] Fig. 11B is a cross-sectional view (XIB-XIB sectional view) of the catheter shaft illustrated in Fig. 10.
[Fig. 11C] Fig. 11C is a cross-sectional view (XIC-XIC sectional view) of the catheter shaft illustrated in Fig. 10.
[Fig. 12] Fig. 12 is a sectional view schematically illustrating a state in which a lead of a ring-shaped electrode passes through a side hole and enters a lumen of the catheter shaft in the electrode catheter illustrated in Fig. 9.

### Description of Embodiments

### <First Embodiment>

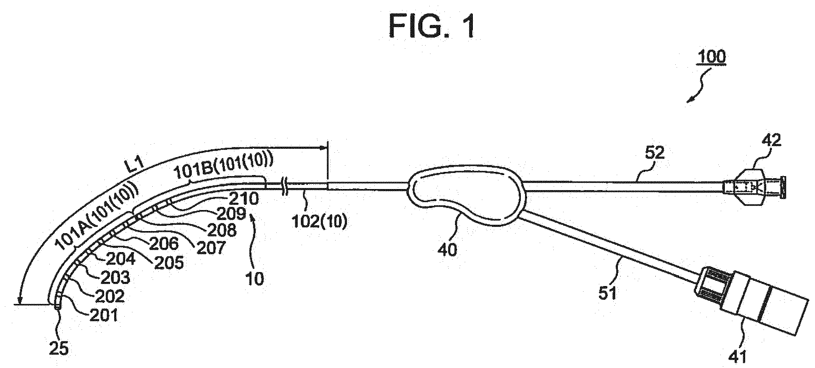

An electrode catheter 100 of the present embodiment illustrated in Figs. 1 to 5 is used to measure an electric potential of a part, such as a coronary artery, of the heart.

The electrode catheter 100 comprises a catheter shaft 10; ring-shaped electrodes 201 to 210 that are attached to the outer circumferential surface of a distal end part 101 of the catheter shaft 10; and leads 301 to 310 that are coupled to the respective ring-shaped electrodes 201 to 210, in which the catheter shaft 10 is constituted by an inside tube 11 that has a guide-wire lumen 11L and an outside tube 13 that forms, in cooperation with the outer circumferential surface of the inside tube 11, a lumen 12L into which the leads 301 to 310 are inserted, the outside tube 13 being configured by a braided tube that is reinforced by a braid 135 made of a resin throughout the entire length thereof; in which side holes 15 that extend from the outer circumferential surface to the lumen 12L are formed in the tube wall of the outside tube 13 at the distal end part 101 of the catheter shaft 10 in correspondence to the attachment positions of the ring-shaped electrodes 201 to 210; and in which the leads 301 to 310 are joined at the respective distal end portions to the inner circumferential surfaces of the ring-shaped electrodes 201 to 210 and are thereby coupled to said ring-shaped electrodes 201 to 210 while entering said lumen 12L through the side holes 15 and extending in the lumen 12L.

In Fig. 1, 25 denotes a distal end tip that is attached to the distal end side of the catheter shaft 10, 40 denotes a handle that is coupled to the proximal end side of the catheter shaft 10, 41 denotes a connector that incorporates a plurality of terminals to which the respective proximal end portions of the leads 301 to 310 are coupled, 42 denotes a guide-wire port, 51 denotes a lead protection tube that has a single-lumen structure that contains the leads 301 to 310 extending out from the handle 40 toward the connector 41, and 52 denotes a guide-wire protection tube that has a single-lumen structure that contains the inside tube 11 extending out from the handle 40 toward the guide-wire port 42.

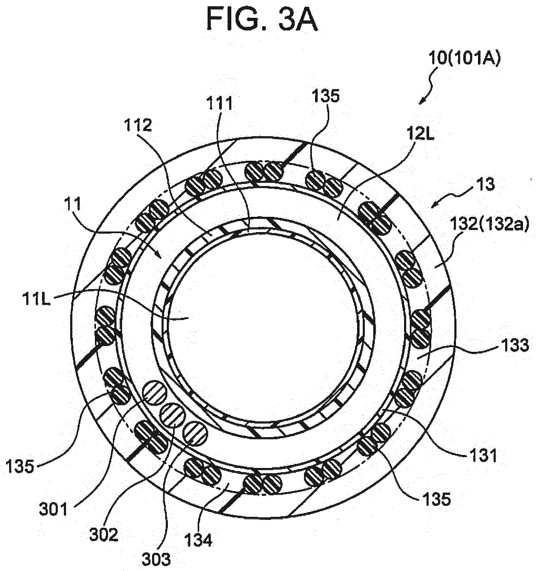

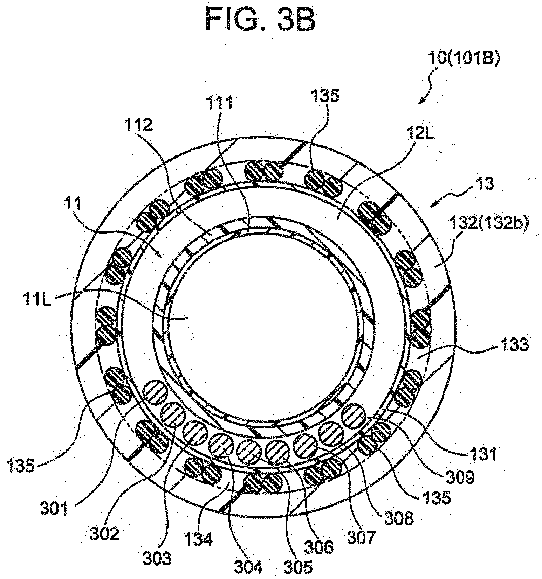

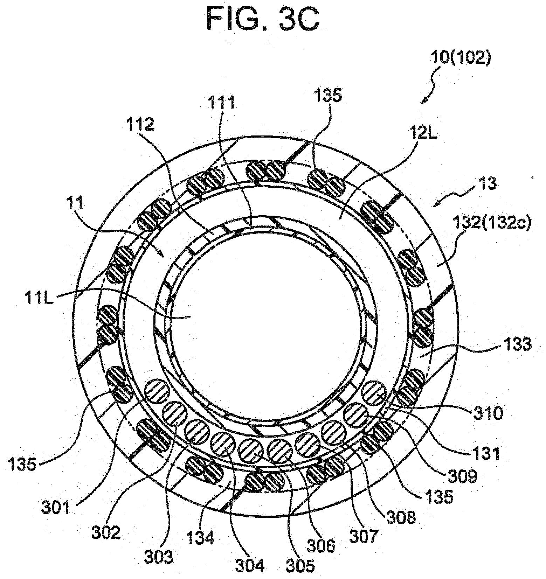

As illustrated in Figs. 3A to 3C, the catheter shaft 10 that constitutes the electrode catheter 100 is a shaft that has a double tube structure constituted by the inside tube 11 and the outside tube 13.

In addition, the catheter shaft 10 is configured by the distal end part 101 and a proximal end part 102, and the distal end part 101 is configured by a distal end-side low hardness region 101A and a hardness gradient region 101B.

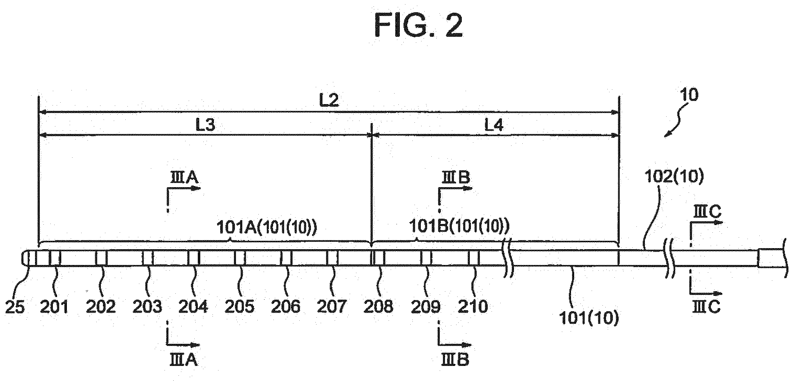

As illustrated in Fig. 1, the distal end part 101 of the catheter shaft 10 has a specific curved shape. Note that, in Fig. 2, the distal end part 101, which actually has the curved shape, is linearly illustrated.

The distal end part 101, which has (memorizes) the specific curved shape, is easily deformable by applying external force (for example, inserting the catheter shaft 10 into the tube) and is, however, restorable to the memorized curved shape when the external force is removed.

The effective length (L1) of the catheter shaft 10 is typically 400 to 1500 mm, and a suitable example thereof is 650 mm.

In addition, the length (L2) of the distal end part 101 is typically 50 to 200 mm, and a suitable example thereof is 95 mm.

In addition, the length (L3) of the distal end-side low hardness region 101A of the distal end part 101 is typically 20 to 100 mm, and a suitable example thereof is 45 mm.

In addition, the length (L4) of the hardness gradient region 101B of the distal end part 101 is typically 20 to 100 mm, and a suitable example thereof is 50 mm.

The inside tube 11 that constitutes the catheter shaft 10 is a single-lumen tube that has a two-layer constitution configured by an inner layer 111 and an outer layer 112, and the guide-wire lumen 11L is formed by the inside tube 11.

Here, as a constituent material of the inner layer 111, a high-density polyethylene or the like is usable. In addition, as a constituent material of the outer layer 112, a polyether block amide (PEBAX) or the like is usable.

The inner diameter of the inside tube 11 is typically 0.5 to 1.5 mm, and a suitable example thereof is 1.0 mm.

The outer diameter of the inside tube 11 is typically 0.6 to 1.7 mm, and a suitable example thereof is 1.2 mm.

The outside tube 13 that constitutes the catheter shaft 10 is configured by the braided tube, which is a resin tube reinforced by the braid 135 made of the resin.

The braid 135 made of the resin, which is a reinforcing material, is embedded in the outside tube 13 throughout the entire length (the distal end part 101 and the proximal end part 102) of the catheter shaft 10.

As illustrated in Figs. 3A to 3C, the outside tube 13 is a single-lumen tube that has a three-layer constitution configured by an inner layer 131, a reinforcing layer 133 configured by the braid 135, and an outer layer 132, and the lumen 12L is formed by the inner circumferential surface of the outside tube 13 and the outer circumferential surface of the inside tube 11.

As a constituent material (inner surface resin) of the inner layer 131 of the outside tube 13, a synthetic resin such as a polyolefin, a polyamide, a polyether polyamide, a polyurethane, a nylon, or a polyether block amide (PEBAX) is usable; among these, PEBAX is preferably used.

Here, the constituent material of the inner layer 131 has the same hardness throughout the entire length of the catheter shaft 10. The hardness of the constituent material of the inner layer 131 is, for example, 72D.

The wall thickness of the inner layer 131 is typically 10 to 50 µm, and a suitable example thereof is 25 µm.

In addition, as a constituent material (outer surface resin) of the outer layer 132 of the outside tube 13, resins of the same type as that of the constituent resin of the inner layer 131 are usable; among these, PEBAX is preferably used.

The constituent material of the outer layer 132 has a hardness that is different along the length direction of the catheter shaft 10. For example, the hardness of the outer layer 132 (132a) that constitutes the distal end-side low hardness region 101A of the distal end part 101 is 35D, and the hardness of the outer layer 132 (132c) that constitutes the proximal end part 102 is 72D. In addition, the hardness of the outer layer 132 (132b) that constitutes the hardness gradient region 101B of the distal end part 101 gradually varies (decreases) from 72D to 35D toward the distal end direction.

The wall thickness of the outer layer 132 is typically 30 to 200 µm, and a suitable example thereof is 95 µm.

The reinforcing layer 133 of the outside tube 13 is configured by the braid 135 made of the resin and a resin 134 that is packed in gaps of resin wire materials that constitute the braid 135.

In the section views illustrated in Figs. 3A to 3C, the braid 135 is configured by 16 pairs (32 pieces) of the resin wire materials that are disposed at equal angular intervals in the circumferential direction.

The resin 134 that constitutes the reinforcing layer 133 was a portion of the constituent material of the outer layer 132, the portion being melted and flowing into and being packed in the gaps of the resin wire materials during production of the outside tube 13.

A constituent material of the braid 135 (resin wire materials) is selected from resins capable of exhibiting a reinforcing effect by being embedded.

The hardness of the constituent material of the braid 135 is preferably 72D or higher. When the hardness is excessively low, it is sometimes not possible to exhibit a sufficient reinforcing effect and favorable torque transmission, consequently.

In addition, the flexural modulus (ISO178 or JISK7171) of the constituent material of the braid 135 is typically 500 to 19,000 MPa, preferably 2,000 to 7,000 MPa and more preferably 3,500 to 4,200 MPa, and a suitable example thereof is 4,200 MPa.

In addition, to maintain the structure of the braid under heating conditions during manufacture of the braided tube, the melting point of the constituent material of the braid 135 (resin wire materials) is preferably higher than the melting points of the constituent materials of the inner layer 131 and the outer layer 132.

A PEEK resin, a polyimide resin, a polyamide resin, a polyester resin, and the like can be enumerated as a reinforcing resin suitable to constitute the braid 135 (resin wire materials); among these, the PEEK resin is preferable in particular.

The wire diameter of the resin wire materials that constitute the braid 135 is typically 30 to 100 µm, and a suitable example thereof is 60 µm.

In addition, the number of carriers of the braid 135 is typically 8 to 32, and a suitable example thereof is 16.

In addition, the number of ends of the braid 135 is typically 1 to 4, and a suitable example thereof is 2.

The wall thickness of the reinforcing layer 133 is typically 60 to 200 µm, and a suitable example thereof is 120 µm.

The inner diameter of the outside tube 13 is typically 0.7 to 2.0 mm, and a suitable example thereof is 1.5 mm.

The outer diameter of the outside tube 13 is typically 1.3 to 3.0 mm, and a suitable example thereof is 2.0 mm.

It is possible to manufacture the outside tube 13, which is a braided tube, by disposing the braid 135 on the outer circumferential surface of an inner-layer formation material that has a tubular shape, disposing an outer-layer formation material on the outer circumferential surface of the braid 135, and subjecting a thus obtained tubular stacked body (laminate) to heat treatment under temperature conditions higher than or equal to the melting points of the inner-layer formation material and the outer-layer formation material and less than the melting point of the resin that constitutes the braid 135.

As illustrated in Figs. 1 and 2, the ring-shaped electrodes 201 to 210 that constitute the electrode catheter 100 are attached to the outer circumferential surface of the distal end part 101 (the distal end-side low hardness region 101A and the hardness gradient region 101B) of the catheter shaft 10. The distal end portions of the leads 301 to 310 are each joined to the inner circumferential surfaces of the ring-shaped electrodes 201 to 210.

As a constituent material of the ring-shaped electrodes 201 to 210, a metal, for example, platinum, gold, silver, aluminum, copper, stainless steel, or the like, that has favorable electric conduction is usable. Note that platinum, gold, silver, alloys containing mainly these components, and the like are preferable from the point of view of obtaining favorable imaging characteristics with respect to X-rays.

The width (the length in the axial direction of the catheter shaft 10) of the ring-shaped electrodes 201 to 210 is, for example, 0.5 to 10 mm.

In addition, the inter-electrode distance of the ring-shaped electrodes 201 to 210 is, for example, 1 to 10 mm.

In the tube wall of the outside tube 13 at the distal end part 101 of the catheter shaft 10, the side holes that extend from the outer circumferential surface thereof to the lumen 12L are formed in correspondence to the attachment positions of the ring-shaped electrodes 201 to 210.

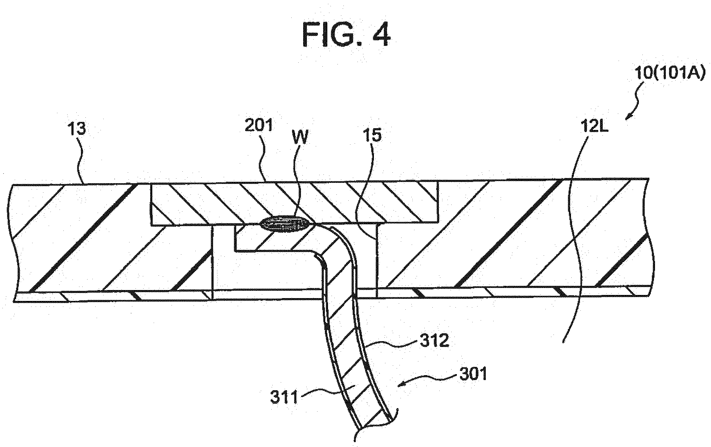

As illustrated in Fig. 4, the lead 301 joined at the distal end portion thereof to the inner circumferential surface of the ring-shaped electrode 201 enters the lumen 12L through the side hole 15 formed in the tube wall of the outside tube 13 and extends in the lumen 12L. In the same figure, 311 denotes a metal core wire of the lead 301, 312 denotes a resin covering layer, and W denotes a joining part. Note that the resin wire materials (not illustrated) that constitute the braid 135 are exposed on the inner circumferential surface of the side hole 15.

The same applies to the leads 302 to 310 that are joined to the inner circumferential surfaces of the ring-shaped electrodes 202 to 210.

A distal end tip 25 is coupled to the distal end side of the catheter shaft 10.

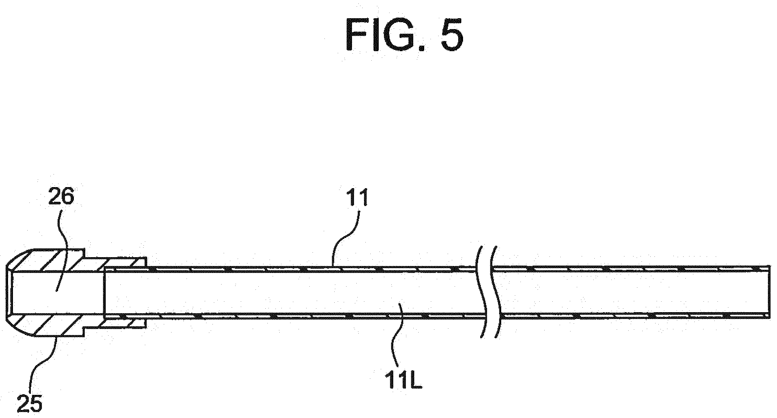

As illustrated in Fig. 5, a through hole 26 is formed in the distal end tip 25, and the distal end portion of the inside tube 11 is inserted into the through hole 26.

The inside tube 11 to which the distal end tip 25 is thus coupled is inserted into the inner part of the outside tube 13, and the catheter shaft 10 that has the double tube structure is thereby constituted.

The proximal end part of the catheter shaft 10 is inserted into the inner part (insertion path) of the handle 40. The insertion path of the catheter shaft 10 in the inner part of the handle 40 branches in two directions, and in the inner part of the handle 40, the leads 301 to 310 extending in the lumen 12L of the catheter shaft 10 each extend out from the proximal end of the outside tube 13, extend along one of the branch paths, extend out from the handle 40, pass through a lumen of the lead protection tube 51, and are inserted into the inner part of the connector 41, and the respective proximal end portions of the leads 301 to 310 are coupled to the plurality of respective terminals incorporated in the connector 41.

Consequently, the ring-shaped electrodes 201 to 210 are each electrically coupled to the respective terminals inside the connector 41.

Meanwhile, in the inner part of the handle 40, the inside tube 11 that constitutes the catheter shaft 10 extends out from the proximal end of the outside tube 13, extends along the other branch path, extends out from the handle 40, passes through a lumen of the guide-wire protection tube 52, and is coupled to the guide-wire port 42.

Consequently, it is possible to cause a guide wire that is inserted through the guide-wire port 42 to extend out from a distal end opening of the distal end tip 25 via the guide-wire lumen 11L of the inside tube 11.

According to the electrode catheter 100 of the present embodiment, the outside tube 13 that constitutes said catheter shaft 10 is configured by the braided tube reinforced by the braid 135 throughout the entire length of the catheter shaft 10, and thus, despite the low ratio (high ratio of the space due to the presence of the guide-wire lumen 11L) of the resin that constitutes said catheter shaft 10, it is possible to exhibit excellent torque transmission, as is clear from the results of the examples, which will be described later.

In addition, the braid 135 that reinforces the outside tube 13, which is the braided tube, is formed of the resin wire materials; therefore, it is possible to easily form, with respect to the tube wall of said outside tube 13 at the distal end part 101, the side holes 15 through which the leads of the ring-shaped electrodes pass because the outside tube 13 is constituted by only a resin material.

In addition, the resin wire materials that constitute the braid 135 are unavoidably exposed on the inner circumferential surfaces of the side holes 15; however, differently from a case in which metal wire materials are exposed, the resin covering layers of the leads inserted into said side holes 15 are prevented from being damaged by the resin wire materials.

In addition, regarding shaping (imparting a bending tendency) of the distal end part of the catheter shaft, which is sometimes performed by an operator when the electrode catheter is used, according to the electrode catheter 100 of the present embodiment, the braid 135 is embedded in the outside tube 13 at the distal end part 101 of the catheter shaft 10, and thus, the bending tendency is easily imparted to said distal end part 101; consequently, it is possible to exhibit favorable shaping characteristics.

### <Second Embodiment>

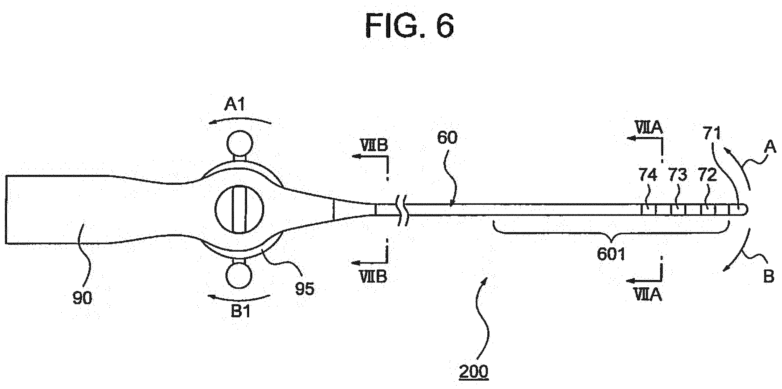

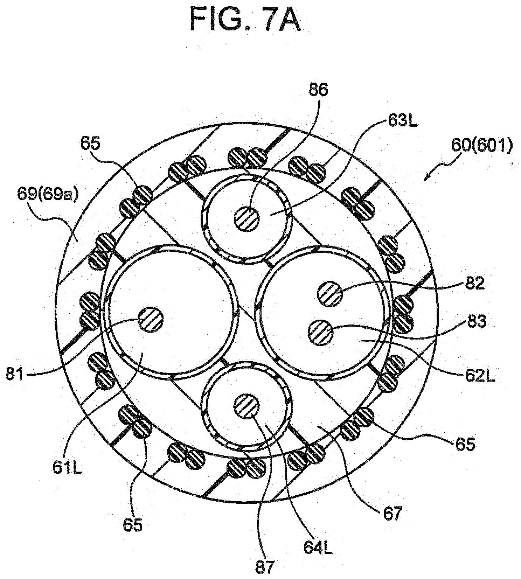

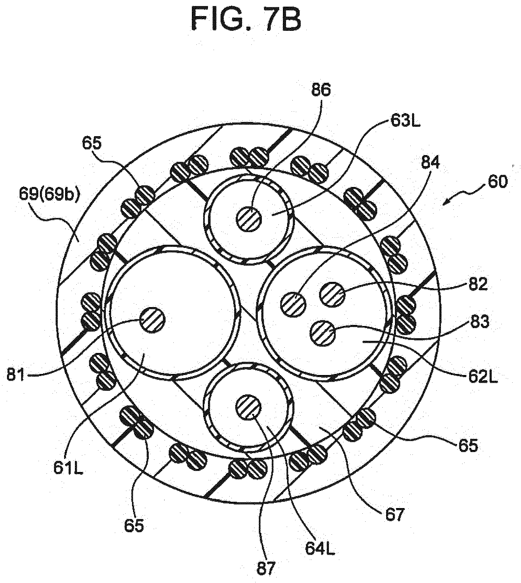

Fig. 6 is a side view illustrating an electrode catheter according to a second embodiment of the present invention, and each of Figs. 7A and 7B is a cross-sectional view of a catheter shaft that constitutes the electrode catheter.

An electrode catheter 200 of the present embodiment comprises a catheter shaft 60 that has a distal end flexible part 601 and that has a multi-lumen structure in which lumens 61L to 64L are formed; a distal end electrode 71 that is coupled to the distal end side of the catheter shaft 60; ring-shaped electrodes 72 to 74 that are attached to the outer circumferential surface of the distal end flexible part 601 of the catheter shaft 60; a lead 81 that is coupled to the distal end electrode 71; leads 82 to 84 that are coupled to the respective ring-shaped electrodes 72 to 74; a first operating wire 86 in which the proximal end thereof is operable by pulling to flex the distal end flexible part 601 of the catheter shaft 60 in a first direction (the direction indicated by the arrow A in Fig. 6); a second operating wire 87 in which the proximal end thereof is operable by pulling to flex the distal end flexible part 601 of the catheter shaft 60 in a second direction (the direction indicated by the arrow B in Fig. 6); and a control handle 90 that is attached to the proximal end side of the catheter shaft 60, in which side holes that extend from the outer circumferential surface to the lumen 62L are formed in the tube wall of the distal end flexible part 601 of the catheter shaft 60 in correspondence to the attachment positions of the ring-shaped electrodes 72 to 74; in which the leads 81 to 84 are configured by coating metal core wires with a resin; in which the lead 81 of the distal end electrode 71 extends in the lumen 61L; in which the leads 82 to 84 of the ring-shaped electrodes 72 to 74 are joined at each of the distal end portions thereof to the inner circumferential surfaces of the ring-shaped electrodes 72 to 74 and are thereby coupled to said ring-shaped electrodes 72 to 74 while entering the lumen 62L through the side holes and extending in said lumen 62L; in which the first operating wire 86 is fixed at the distal end portion thereof to the distal end electrode 71 while extending in the lumen 63L; in which the second operating wire 87 is fixed at the distal end portion thereof to the distal end electrode 71 while extending in the lumen 64L; and in which the catheter shaft 60 is configured by a braided tube that is reinforced by a braid 65 made of a resin throughout the entire length thereof.

In Fig. 6, 95 denotes a knob for performing swinging operation (distal-end deflection operation of the catheter) of the catheter shaft 60.

In addition, in Figs. 7A and 7B, 67 denotes an inner (core) portion that is configured by a resin and that constitutes the catheter shaft 60, and 69 denotes an outer (shell) portion that is configured by a resin and that covers the inner portion 67.

As illustrated in Fig. 6, the electrode catheter 200 comprises the catheter shaft 60, the distal end electrode 71 fixed to the distal end of the catheter shaft 60, the ring-shaped electrodes 72 to 74 attached to the distal end flexible part of the catheter shaft 60, and the control handle 90 attached to the proximal end side of the catheter shaft 60.

The distal end part of the catheter shaft 60 is the distal end flexible part 601. Here, the "distal end flexible part" means a distal end part of the catheter shaft that is flexible (bendable) by pulling operation of an operating wire (the first operating wire 86 or the second operating wire 87).

The outer diameter of the catheter shaft 60 is typically 0.6 to 3 mm and preferably 1.3 to 2.4 mm.

The length of the catheter shaft 60 is typically 400 to 1500 mm and preferably 700 to 1200 mm.

The length of the distal end flexible part 601 is, for example, 30 to 300 mm and preferably 50 to 250 mm.

The control handle 90 is attached to the proximal end side of the catheter shaft 60. A connector that comprises a plurality of terminals is disposed in the control handle 90, and the proximal end portions of the leads 81 and 82 to 84 respectively coupled to the distal end electrode 71 and the ring-shaped electrodes 72 to 74 are coupled to the terminals of the connector.

In addition, the knob 95 for performing bending operation of the distal end flexible part 601 of the catheter shaft 60 is attached to the control handle 90.

As illustrated in Figs. 7A and 7B, the catheter shaft 60 comprises the inner portion 67 configured by the resin, the outer portion 69 configured by the resin and covering the inner portion 67, and the braid 65 made of the resin, the braid 65 being embedded in the inner part of the outer portion 69 throughout the entire length of the catheter shaft 60, the catheter shaft 60 being a braided tube that has the multi-lumen structure having the four lumens 61L to 64L inside the braid 65 (inner portion 67).

A thermoplastic polyamide elastomer can be enumerated as a resin that constitutes the inner portion 67; in particular, a polyether block amide (PEBAX) is preferable.

The hardness of the resin that constitutes the inner portion 67 is, for example, 25D to 40D.

The first operating wire 86 and the second operating wire 87 are each inserted into the lumen 63L and the lumen 64L that have relatively small diameters and that are disposed opposite to each other with the center axis of the catheter shaft 60 therebetween.

The lumen 61L and the lumen 62L are lumens that have relatively large diameters and that are disposed opposite to each other with the center axis of the catheter shaft 60 therebetween.

The lead 81 coupled to the distal end electrode 71 is inserted into the lumen 61L, and the leads 82 to 84 coupled to the respective ring-shaped electrodes 72 to 74 are inserted into the lumen 62L.

The lead 81 is coupled and fixed at the distal end portion thereof to the distal end electrode 71 by solder packed in the internal space of the distal end electrode 71, and is thereby coupled to said distal end electrode 71.

The leads 82 to 84 are joined at each of the distal end portions thereof to the inner circumferential surfaces of the ring-shaped electrodes 72 to 74 and are thereby coupled to said ring-shaped electrodes 72 to 74 while entering the lumen 62L through the side holes formed in the tube wall of the distal end flexible part 601 of the catheter shaft 60 and being inserted into said lumen 62L.

The outer portion 69 is configured by a resin material that covers the inner portion 67.

A thermoplastic polyamide elastomer can be enumerated as the resin material that constitutes the outer portion 69; in particular, a polyether block amide (PEBAX) is preferable.

Note that the outer portion 69 may be constituted by a tube having physical properties that are the same along the axial direction; however, preferably, the hardness varies (decreases) toward the distal end direction gradually or step by step. For example, the hardness of the outer portion 69 (69a) illustrated in Fig. 7A is, for example, 25D to 55D, and the hardness of the outer portion 69 (69b) illustrated in Fig. 7B is 55D to 72D.

As illustrated in Figs. 7A and 7B, the braid 65 made of the resin, which is a reinforcing material, is embedded in the inner part of the outer portion 69.

In the section views illustrated in Figs. 7A and 7B, the braid 65 is configured by 16 pairs (32 pieces) of resin wire materials that are disposed at equal angular intervals in the circumferential direction.

The constituent material of the braid 65 (resin wire materials), the hardness of the constituent material, the wire diameter of the resin wire materials, and the like are the same as those of the braid 135 in the electrode catheter 100 of the first embodiment.

The electrode catheter 200 of the present embodiment comprises the first operating wire 86 for flexing the distal end flexible part 601 of the catheter shaft 60 in the first direction (the direction indicated by the arrow A) and the second operating wire 87 for flexing the distal end flexible part 601 of the catheter shaft 60 in the second direction (the direction indicated by the arrow B).

The first operating wire 86 is inserted so as to be movable in the tube axis direction in the inner part (lumen 63L) of the catheter shaft 60. The distal end of the first operating wire 86 is coupled and fixed to the distal end electrode 71 by solder packed in the internal space of the distal end electrode 71. In addition, the proximal end of the first operating wire 86 is coupled to the knob 95 of the control handle 90 and is thereby operable by pulling.

Meanwhile, the second operating wire 87 is inserted so as to be movable in the tube axis direction in the inner part (lumen 64L) of the catheter shaft 60. The distal end of the second operating wire 87 is coupled and fixed to the distal end electrode 71 by solder, similarly to the first operating wire 86. In addition, the proximal end of the second operating wire 87 is coupled to the knob 95 of the control handle 90 and is thereby operable by pulling.

When the knob 95 of the control handle 90 that constitutes the electrode catheter 200 is rotated in the A1 direction illustrated in Fig. 6, the first operating wire 86 is pulled and moved to the proximal end side of the lumen 63L, and the distal end flexible part 601 can be flexed in the first direction (the direction indicated by the arrow A).

Meanwhile, when the knob 95 of the control handle 90 is rotated in the B1 direction illustrated in Fig. 6, the second operating wire 87 is pulled and moved to the proximal end side of the lumen 64L, and the distal end flexible part 601 can be flexed in the second direction (the direction indicated by the arrow B).

Moreover, orientations in the first direction and the second direction with respect to the catheter 200 can be freely set in a state inserted in a body cavity by rotating the control handle 90 around the axis.

According to the electrode catheter 200 of the present embodiment, the braid 65 made of the resin is embedded in the inner part of the outer portion 69 throughout the entire length of the catheter shaft 60, and thus, it is possible to exhibit excellent torque transmission.

In addition, the braid 65 that reinforces the catheter shaft 60 is formed of the resin wire materials; therefore, it is possible to easily form, with respect to the tube wall of the distal end flexible part 601, the side holes through which the leads 82 to 84 of the ring-shaped electrodes 72 to 74 pass because the catheter shaft 60 is constituted by only a resin material.

In addition, the resin wire materials that constitute the braid 65 and that are exposed on the inner circumferential surfaces of the formed side holes are prevented from damaging the resin covering layers of the leads 82 to 84 inserted into said side holes.

### Further Example

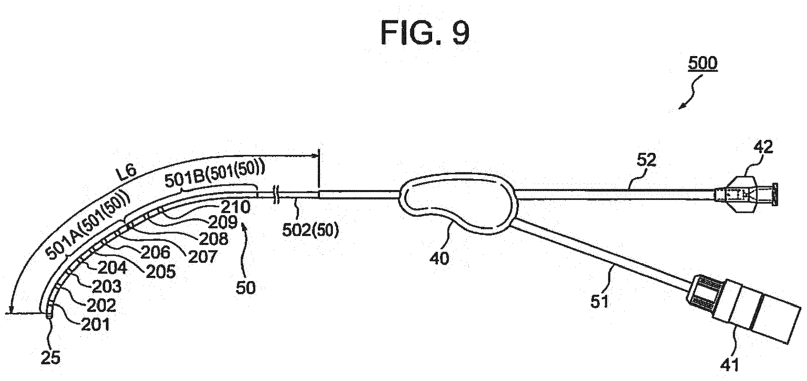

An electrode catheter 500 of the example illustrated in Figs. 9 to 12 is used to measure an electric potential of a part, such as a coronary artery, of the heart.

Note that, in the Figs. 9 to 12, portions denoted by the same reference signs as in Figs. 1 to 4 have the same constitutions as those of the electrode catheter of the first embodiment. This example is not part of the present invention as the catheter according to this example does not comprise a resin braid throughout the entire length of the catheter.

The electrode catheter 500 of the present example comprises a catheter shaft 50; the ring-shaped electrodes 201 to 210 that are attached to the outer circumferential surface of a distal end part 501 of the catheter shaft 50; and the leads 301 to 310 that are coupled to the respective ring-shaped electrodes 201 to 210, in which the catheter shaft 50 is constituted by the inside tube 11 that has the guide-wire lumen 11L and an outside tube 53 that forms, in cooperation with the outer circumferential surface of the inside tube 11, the lumen 12L into which the leads 301 to 310 are inserted; in which the outside tube 53 that constitutes the catheter shaft 50 is configured by a braided tube that has a distal end part 531 reinforced by a braid 5315 made of a resin and a proximal end part 532 reinforced by a braid 5325 made of a metal; in which the side holes 15 that extend from the outer circumferential surface to the lumen 12L are formed in the tube wall at the distal end part 531 of the outside tube 53 in correspondence to the attachment positions of the ring-shaped electrodes 201 to 210; and in which the leads 301 to 310 are joined at each of the distal end portions thereof to the inner circumferential surfaces of the ring-shaped electrodes 201 to 210 and are thereby coupled to said ring-shaped electrodes 201 to 210 while entering the lumen 12L through the side holes 15 and extending in said lumen 12L.

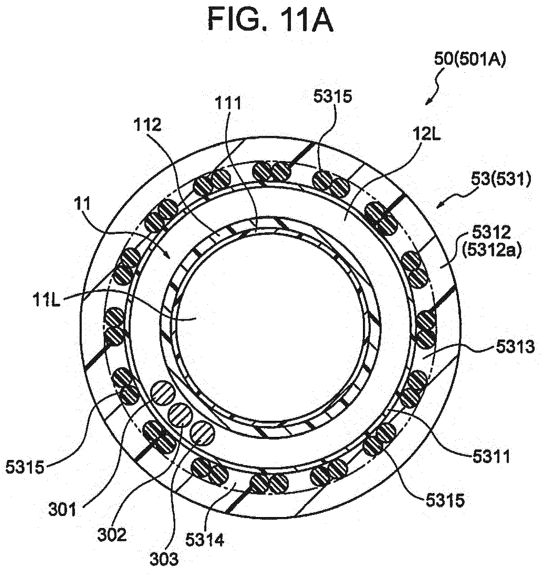

As illustrated in Figs. 11A to 11C, the catheter shaft 50 that constitutes the electrode catheter 500 is a shaft that has a double tube structure constituted by the inside tube 11 and the outside tube 53.

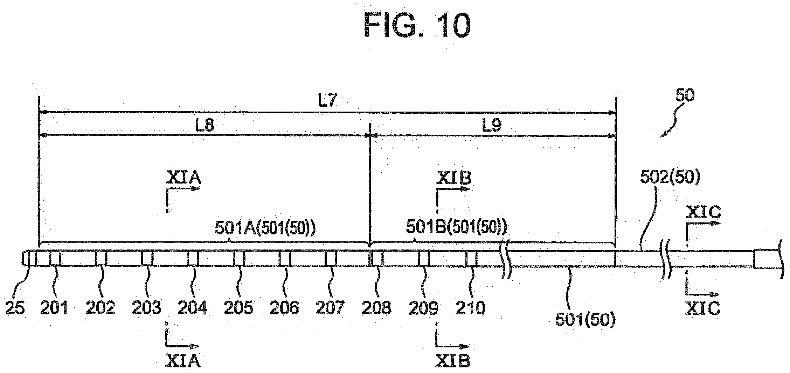

In addition, as illustrated in Figs. 9 and 10, the catheter shaft 50 is configured by the distal end part 501 and a proximal end part 502, and the distal end part 501 is configured by a distal end-side low hardness region 501A and a hardness gradient region 501B.

As illustrated in Fig. 9, the distal end part 501 of the catheter shaft 50 has a specific curved shape. Note that, in Fig. 10, the distal end part 501, which actually has the curved shape, is linearly illustrated.

The distal end part 501, which has (memorizes) the specific curved shape, is easily deformable by applying external force (for example, inserting the catheter shaft 50 into the tube) and is, however, restorable to the memorized curved shape when the external force is removed.

The effective length (L6) of the catheter shaft 50 is typically 400 to 1500 mm, and a suitable example thereof is 650 mm.

In addition, the length (L7) of the distal end part 501 is typically 50 to 200 mm, and a suitable example thereof is 95 mm.

In addition, the length (L8) of the distal end-side low hardness region 501A of the distal end part 501 is typically 20 to 100 mm, and a suitable example thereof is 45 mm.

In addition, the length (L9) of the hardness gradient region 501B of the distal end part 501 is typically 20 to 100 mm, and a suitable example thereof is 50 mm.

The inside tube 11 that constitutes the catheter shaft 50 is a single-lumen tube that has a two-layer constitution configured by the inner layer 111 and the outer layer 112, and the guide-wire lumen 11L is formed by the inside tube 11.

The specific constitution of the inside tube 11 is the same as that of the inside tube 11 that constitutes the catheter shaft 10 of the electrode catheter of the first embodiment.

The outside tube 53 that constitutes the catheter shaft 50 is configured by the braided tube that has the distal end part 531 reinforced by the braid 5315 made of the resin and the proximal end part 532 reinforced by the braid 5325 made of the metal.

That is, the braid 5315 made of the resin is embedded as a reinforcing material in the distal end part 531 of the outside tube 53, and the braid 5325 made of the metal is embedded as a reinforcing material in the proximal end part 532 of the outside tube 53.

Moreover, the distal end part 531 of the outside tube 53 constitutes the distal end part 501 of the catheter shaft 50, and the proximal end part 532 of the outside tube 53 constitutes the proximal end part 502 of the catheter shaft 50.

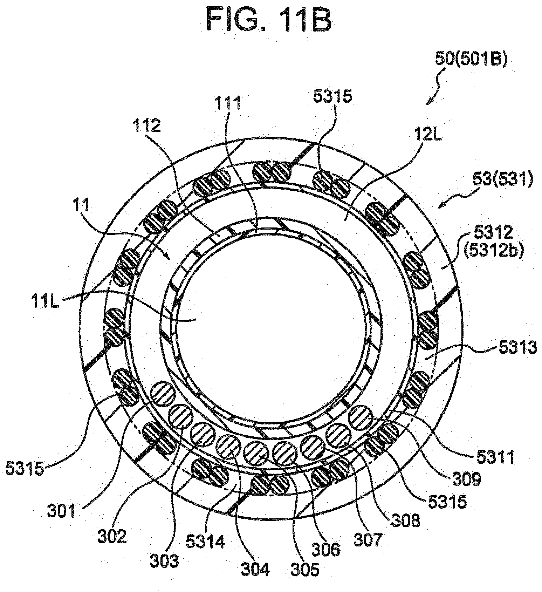

As illustrated in Figs. 11A and 11B, the distal end part 531 of the outside tube 53 is a single-lumen tube that has a three-layer constitution configured by an inner layer 5311, a reinforcing layer 5313 configured by the braid 5315, and an outer layer 5312.

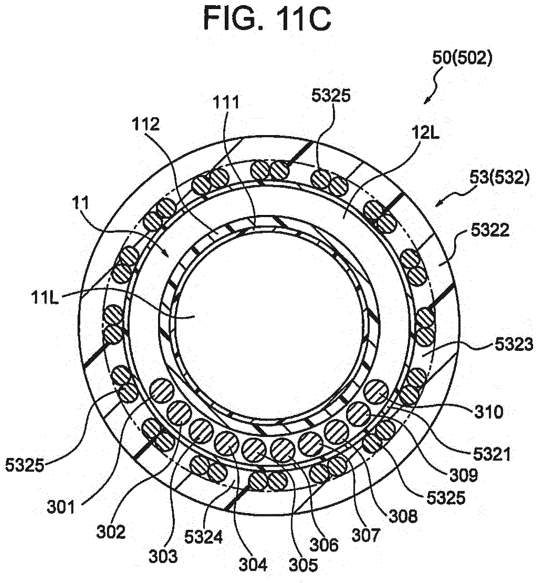

In addition, as illustrated in Fig. 11C, the proximal end part 532 of the outside tube 53 is a single-lumen tube that has a three-layer constitution configured by an inner layer 5321, a reinforcing layer 5323 configured by the braid 5325, and an outer layer 5322.

The lumen 12L is formed by the inner circumferential surface of the outside tube 53 (the distal end part 531 and the proximal end part 532) and the outer circumferential surface of the inside tube 11.

As a constituent material (inner surface resin) of the inner layer 5311, 5321 of the outside tube 53, a synthetic resin such as a polyolefin, a polyamide, a polyether polyamide, a polyurethane, a nylon, or a polyether block amide (PEBAX) is usable; among these, PEBAX is preferably used.

Here, the constituent materials of the inner layer 5311, 5321 has the same hardness throughout the entire length of the catheter shaft 50. The hardness of the constituent material of the inner layer 5311, 5321 is, for example, 72D.

The wall thickness of the inner layer 5311, 5321 is typically 10 to 50 µm, and a suitable example thereof is 25 µm.

In addition, as a constituent material (outer surface resin) of the outer layer 5312, 5322 of the outside tube 53, resins of the same type as that of the constituent resin of the inner layer 5311, 5321 are usable; among these, PEBAX is preferably used.

The constituent material of the outer layer 5312, 5322 has a hardness that is different along the length direction of the catheter shaft 50. For example, the hardness of the outer layer 5312 (5312a) that constitutes the distal end-side low hardness region 501A of the distal end part 501 is 35D, and the hardness of the outer layer 5322 that constitutes the proximal end part 502 is 72D. In addition, the hardness of the outer layer 5312 (5312b) that constitutes the hardness gradient region 501B of the distal end part 501 gradually varies (decreases) from 72D to 35D toward the distal end direction.

The wall thickness of the outer layer 5312, 5322 is typically 30 to 200 µm, and a suitable example thereof is 95 µm.

The reinforcing layer 5313 at the distal end part 531 of the outside tube 53 is configured by the braid 5315 made of the resin and a resin 5314 that is packed in gaps of resin wire materials that constitute the braid 5315.

In addition, the reinforcing layer 5323 at the proximal end part 532 of the outside tube 53 is configured by the braid 5325 made of the metal and a resin 5324 that is packed in gaps of metal wire materials that constitute the braid 5325.

In the section views illustrated in Figs. 11A and 11B, the braid 5315 is configured by 16 pairs (32 pieces) of the resin wire materials that are disposed at equal angular intervals in the circumferential direction.

The resin 5314 that constitutes the reinforcing layer 5313 was a portion of the constituent material of the outer layer 5312, the portion being melted and flowing into and being packed in the gaps of the resin wire materials during production of the distal end part 531 of the outside tube 53.

The constituent material of the braid 5315 (resin wire materials), the hardness and the flexural modulus of the constituent material, the wire diameter of the resin wire materials, the number of carriers and the number of ends of the braid 5315, and the like are the same as those of the braid 135 in the electrode catheter 100 of the first embodiment.

The wall thickness of the reinforcing layer 5313 is typically 60 to 200 µm, and a suitable example thereof is 120 µm.

In the section view illustrated in Fig. 11C, the braid 5325 is configured by 16 pairs (32 pieces) of the metal wire materials that are disposed at equal angular intervals in the circumferential direction.

The resin 5324 that constitutes the reinforcing layer 5323 was a portion of the constituent material of the outer layer 5322, the portion being melted and flowing into and being packed in the gaps of the resin wire materials during production of the proximal end part 532 of the outside tube 53.

The constituent material of the braid 5325 (metal wire materials) is not particularly limited, and all metal materials used in the braided tube that constitutes the catheter shaft are usable; an example thereof is a stainless steel element wire or the like.

The wire diameter of the metal wire materials that constitute the braid 5325 is typically 30 to 100 µm, and a suitable example thereof is 60 µm.

In addition, the number of carriers of the braid 5325 is typically 8 to 32, and a suitable example thereof is 16.

In addition, the number of ends of the braid 5325 is typically 1 to 4, and a suitable example thereof is 2.

The wall thickness of the reinforcing layer 5323 is typically 60 to 200 µm, and a suitable example thereof is 120 µm.

The inner diameter of the outside tube 53 is typically 0.7 to 2.0 mm, and a suitable example thereof is 1.5 mm.

The outer diameter of the outside tube 53 is typically 1.3 to 3.0 mm, and a suitable example thereof is 2.0 mm.

It is possible to manufacture the distal end part 531 of the outside tube 53 by disposing the braid 5315 made of the resin on the outer circumferential surface of an inner-layer formation material that has a tubular shape, disposing an outer-layer formation material on the outer circumferential surface of the braid 5315, and subjecting a thus obtained tubular stacked body to heat treatment under temperature conditions higher than or equal to the melting points of the inner-layer formation material and the outer-layer formation material and less than the melting point of the resin that constitutes the braid 5315.

It is possible to manufacture the proximal end part 532 of the outside tube 53 by disposing the braid 5325 made of the metal on the outer circumferential surface of an inner-layer formation material that has a tubular shape, disposing an outer-layer formation material on the outer circumferential surface of the braid 5325, and subjecting a thus obtained tubular stacked body to heat treatment under temperature conditions higher than or equal to the melting points of the inner-layer formation material and the outer-layer formation material.

It is possible to manufacture the outside tube 53 by joining the distal end part 531 and the proximal end part 532 that are obtained as described above to each other by a conventional method.

As illustrated in Figs. 9 and 10, the ring-shaped electrodes 201 to 210 that constitute the electrode catheter 500 are attached to the outer circumferential surface of the distal end part 501 (the distal end-side low hardness region 501A and the hardness gradient region 501B) of the catheter shaft 50. The distal end portions of the leads 301 to 310 are each joined to the inner circumferential surfaces of the ring-shaped electrodes 201 to 210.

As a constituent material of the ring-shaped electrodes 201 to 210, a metal, for example, platinum, gold, silver, aluminum, copper, or stainless steel, having favorable electric conduction is usable. Note that platinum, gold, silver, alloys containing mainly these components, and the like are preferable from the point of view of obtaining favorable imaging characteristics with respect to X-rays.

The width (the length in the axial direction of the catheter shaft 50) of the ring-shaped electrodes 201 to 210 is, for example, 0.5 to 10 mm.

In addition, the inter-electrode distance of the ring-shaped electrodes 201 to 210 is, for example, 1 to 10 mm.

In the tube wall of the outside tube 53 at the distal end part 531 of the catheter shaft 50, the side holes 15 that extend from the outer circumferential surface thereof to the lumen 12L are formed in correspondence to the attachment positions of the ring-shaped electrodes 201 to 210.

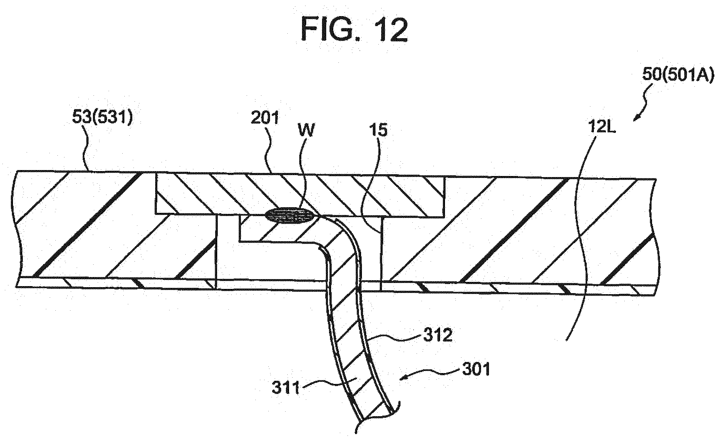

As illustrated in Fig. 12, the lead 301 that is joined at the distal end portion thereof to the inner circumferential surface of the ring-shaped electrode 201 enters the lumen 12L through the side hole 15 that is formed in the tube wall of the outside tube 53 and extends in the lumen 12L. In the same figure, 311 denotes the metal core wire of the lead 301, 312 denotes the resin covering layer, and W denotes the joining part. Note that the resin wire materials (not illustrated) that constitute the braid 5315 are exposed on the inner circumferential surface of the side hole 15.

The same applies to the leads 302 to 310 that are joined to the inner circumferential surfaces of the ring-shaped electrodes 202 to 210.

As illustrated in Figs. 9 and 10, the distal end tip 25 is coupled to the distal end side of the catheter shaft 50.

A through hole is formed in the distal end tip 25, and the distal end portion of the inside tube 11 is inserted into the through hole.

The inside tube 11 to which the distal end tip 25 is thus coupled is inserted into the inner part of the outside tube 53, and the catheter shaft 50 that has the double tube structure is thereby constituted.

The proximal end part 502 of the catheter shaft 50 is inserted into the inner part (insertion path) of the handle 40. The insertion path of the catheter shaft 50 in the inner part of the handle 40 branches in two directions, and in the inner part of the handle 40, the leads 301 to 310 extending in the lumen 12L of the catheter shaft 50 each extend out from the proximal end of the outside tube 53, extend along one of the branch paths, extend out from the handle 40, pass through a lumen of the lead protection tube 51, and are inserted into the inner part of the connector 41, and the respective proximal end portions of the leads 301 to 310 are coupled to the plurality of respective terminals incorporated in the connector 41.

Consequently, the ring-shaped electrodes 201 to 210 are each electrically coupled to the respective terminals inside the connector 41.

Meanwhile, in the inner part of the handle 40, the inside tube 11 that constitutes the catheter shaft 50 extends out from the proximal end of the outside tube 53, extends along the other branch path, extends out from the handle 40, passes through a lumen of the guide-wire protection tube 52, and is coupled to the guide-wire port 42.

Consequently, it is possible to cause a guide wire that is inserted through the guide-wire port 42 to extend out from the distal end opening of the distal end tip 25 via the guide-wire lumen 11L of the inside tube 11.

According to the electrode catheter 500 of the present example , the outside tube 53 that constitutes the catheter shaft 50 is constituted by the braided tube throughout the entire length including the distal end part 531, and thus, it is possible to exhibit excellent torque transmission.

In addition, the proximal end part 532 of the outside tube 53 is reinforced by the braid 5325 made of the metal, and thus, the torque transmission exhibited by the electrode catheter 500 of the present example is more excellent than the torque transmission of the electrode catheter of the first embodiment.

In addition, the braid 5315 that reinforces the distal end part 531 of the outside tube 53 is formed of the resin wire materials, and thus, it is possible to easily form, with respect to the tube wall of the outside tube 53 at the distal end part 531, the side holes 15 through which the leads of the ring-shaped electrodes pass.

In addition, the resin wire materials that constitute the braid 5315 are unavoidably exposed on the inner circumferential surface of the side holes 15; however, differently from a case in which metal wire materials are exposed, the resin covering layers of the leads inserted into said side holes 15 are prevented from being damaged by the resin wire materials.

In addition, regarding shaping (imparting a bending tendency) of the distal end part of a catheter shaft, which is sometimes performed by an operator when an electrode catheter is used, according to the electrode catheter 500 of the present example, the braid 5315 is embedded in the outside tube 53 at the distal end part 501 of the catheter shaft 50, and thus, the bending tendency is easily imparted to said distal end part 531; consequently, it is possible to exhibit favorable shaping characteristics.

In the foregoing, the embodiments of the present invention have been described; however, the present invention is not limited to these embodiments and can be variously modified.

For example, in the electrode catheters of the first embodiment and the example, the number (the number of the leads) of the ring-shaped electrodes may be, for example, 2 to 30 instead of 10.

In addition, a distal end electrode may be comprised as an alternative to the distal end tip 25.

In addition, in the electrode catheter of the second embodiment, the number of the lumens formed in the distal end flexible part of the catheter shaft may be, for example, eight or more instead of four.

In addition, the number of the operating wires may be one (single direction type).

In addition, the braid that reinforces the distal end part (distal end flexible part) of the catheter shaft may be made of a resin (braid similar to the braid 65), and the braid that reinforces the proximal end part of the catheter shaft may be made of a metal.

### EXAMPLES

### <Example 1>

(1) Production of Inside Tube:
   The inside tube 11 (inner diameter = 1.0 mm, outer diameter = 1.18 mm) that has the two-layer constitution configured by the inner layer 111 that is configured by a high density polyethylene and that has a thickness of 30 µm and the outer layer 112 that is configured by PEBAX (hardness 55D) and that has a thickness of 60 µm was produced.
(2) Production of Outside Tube:
   The outside tube 13 (inner diameter = 1.5 mm, outer diameter = 2.0 mm) that has the three-layer constitution constituted by the inner layer 131 that has a thickness of 25 µm, the reinforcing layer 133 that has a thickness of 120 µm, and the outer layer 132 that has a thickness of 95 µm was produced by disposing the braid 135 (number of carriers = 16, number of ends = 2) configured by PEEK resin wire materials that have a wire diameter of 60 µm on the outer circumferential surface of the inner-layer formation material that is configured by PEBAX (hardness 72D) and that has a tubular shape, disposing the outer-layer formation material that is configured by PEBAX on the outer circumferential surface of the braid 135, and subjecting the thus obtained tubular stacked body to heat treatment.
(3) Manufacture of Electrode Catheter:
   The electrode catheter 100 according to the first embodiment of the present invention, such as those illustrated in Figs. 1 to 5, was manufactured through steps of producing the catheter shaft 10 by using the inside tube 11 obtained according to (1) described above and the outside tube 13 obtained according to (2) described above, attaching the ring-shaped electrodes 201 to 210 to the outer circumferential surface of the distal end part 101 of the obtained catheter shaft 10, and inserting the leads 301 to 310 that are coupled to the respective ring-shaped electrodes 201 to 210 into the lumen 12L through the side holes 15.

In the catheter shaft 10 that constitutes the electrode catheter 100, the effective length (L1) thereof is 650 mm, the length (L3) of the distal end-side low hardness region 101A of the distal end part 101 is 50 mm, the hardness of the outer layer 132 (132a) that constitutes the distal end-side low hardness region 101A is 35D, the length (L4) of the hardness gradient region 101B is 50 mm, the hardness of the outer layer 132 (132b) that constitutes the hardness gradient region 101B gradually varies from 35D to 72D, and the hardness of the outer layer 132 (132c) that constitutes the proximal end part 102 is 72D.

### <Comparative Example 1>

(1) An inside tube is produced similarly to Example 1 (1) .
(2) Production of Outside Tube:
   An outside tube (inner diameter = 1.5 mm, outer diameter = 2.0 mm) configured by the proximal end part constituted by a stainless-steel braided tube and the distal end part constituted by a resin tube that is not reinforced by a braid was produced by disposing a braid (number of carriers = 16, number of ends = 1) that is configured by stainless steel wire materials having a wire diameter of 50 µm on the outer circumferential surface, excluding a part 100 mm from the distal end, of an inner-layer formation material that is configured by PEBAX (hardness 72D) and that has a tubular shape, additionally disposing, throughout the entire length of the inner-layer formation material, an outer-layer formation material that is configured by PEBAX, and subjecting an obtained tubular stacked body to heat treatment.
(3) Manufacture of Electrode Catheter:
   An electrode catheter for comparison purposes was manufactured similarly to Example 1 (3) except for producing a catheter shaft by using the inside tube obtained according to (1) described above and the outside tube obtained according to (2) described above.

In the catheter shaft that constitutes the electrode catheter, the effective length thereof is 650 mm, the hardness of the distal end region (0 to 50 mm from the distal end) of the distal end part 101 is 35D, the hardness of the rear end region (50 to 100 mm from the distal end) of the distal end part 101 is 55D, and the hardness of the proximal end part that is constituted by the stainless steel braided tube is 72D.

### <Evaluation of Torque Transmission>

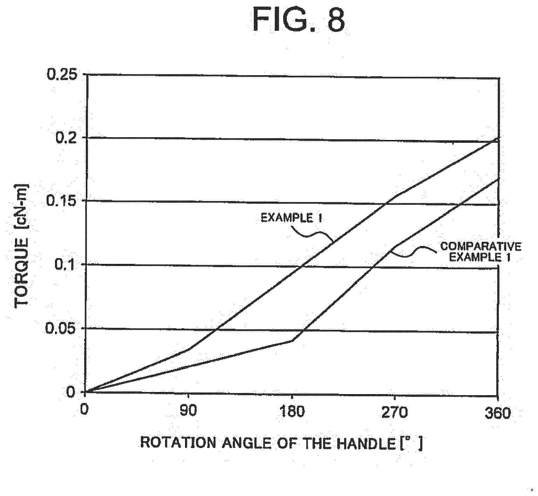

Regarding the respective electrode catheters obtained according to Example 1 and Comparative Example 1, in a state in which the distal end of the catheter shaft is fixed, the handle is rotated around the axis, and torque that is transmitted to the distal end along with the rotation was measured with a torque gauge. Results (the relationship between the rotation angle of the handle and the torque) are indicated in Fig. 8.

As indicated in Fig. 8, it is understood that the electrode catheter obtained according to Example 1 is excellent in terms of torque transmission compared with the electrode catheter (electrode catheter in which the proximal end part of the catheter shaft is constituted by the stainless steel braided tube) obtained according to Comparative Example 1.

### Reference Signs List

- 100: ELECTRODE CATHETER
- 10: CATHETER SHAFT
- 101: DISTAL END PART
- 101A: DISTAL END-SIDE LOW HARDNESS REGION
- 101B: HARDNESS GRADIENT REGION
- 102: PROXIMAL END PART
- 11: INSIDE TUBE
- 111: INNER LAYER
- 112: OUTER LAYER
- 11L: GUIDE-WIRE LUMEN
- 12L: LUMEN
- 13: OUTSIDE TUBE
- 131: INNER LAYER
- 132: OUTER LAYER
- 133: REINFORCING LAYER
- 134: RESIN
- 135: BRAID
- 15: SIDE HOLE
- 201-210: RING-SHAPED ELECTRODE
- 25: DISTAL END TIP
- 301-310: LEAD
- 40: HANDLE
- 41: CONNECTOR
- 42: GUIDE-WIRE PORT
- 51: WIRE PROTECTION TUBE
- 52: GUIDE-WIRE PROTECTION TUBE
- 200: ELECTRODE CATHETER
- 60: CATHETER SHAFT
- 601: DISTAL END FLEXIBLE PART
- 61L-64L: LUMEN
- 65: BRAID
- 67: INNER PORTION
- 69: OUTER PORTION
- 71: DISTAL END ELECTRODE
- 72-74: RING-SHAPED ELECTRODE
- 81-84: LEAD
- 86: FIRST OPERATING WIRE
- 87: SECOND OPERATING WIRE
- 90: CONTROL HANDLE
- 500: ELECTRODE CATHETER
- 50: CATHETER SHAFT
- 501: DISTAL END PART OF CATHETER SHAFT
- 501A: DISTAL END-SIDE LOW HARDNESS REGION
- 501B: HARDNESS GRADIENT REGION
- 502: PROXIMAL END PART OF CATHETER SHAFT
- 53: OUTSIDE TUBE
- 531: DISTAL END PART OF OUTSIDE TUBE
- 5311: INNER LAYER
- 5312: OUTER LAYER
- 5313: REINFORCING LAYER
- 5314: RESIN
- 5315: BRAID MADE OF RESIN
- 532: PROXIMAL END PART OF OUTSIDE TUBE
- 5321: INNER LAYER
- 5322: OUTER LAYER
- 5323: REINFORCING LAYER
- 5324: RESIN
- 5325: BRAID MADE OF METAL

## Claims

1. An electrode catheter (100, 200) comprising:
a catheter shaft (10, 60) that has at least one lumen (12L, 61L, 62L, 63L, 64L); at least one ring-shaped electrode (201, 202, 203, 204, 205, 206, 207, 208, 209, 210; 72, 73, 74) that is attached to an outer circumferential surface of a distal end part (101, 601) of the catheter shaft (10, 60); and a lead (301, 302, 303, 304, 305, 306, 307, 308, 309, 310; 82, 83, 84) that is coupled to the ring-shaped electrode (201, 202, 203, 204, 205, 206, 207, 208, 209, 210; 72, 73, 74),
wherein a side hole (15) that extends from the outer circumferential surface to the lumen (12L) is formed in a tube wall of the distal end part (101, 601) of the catheter shaft (10, 60) in correspondence to an attachment position of the ring-shaped electrode (201, 202, 203, 204, 205, 206, 207, 208, 209, 210,72, 73, 74),
wherein the lead (301, 302, 303, 304, 305, 306, 307, 308, 309, 310; 82, 83, 84) is configured by coating (312) a metal core wire (311) with a resin, the lead (301, 302, 303, 304, 305, 306, 307, 308, 309, 310; 82, 83, 84) being joined at a distal end portion thereof to an inner circumferential surface of the ring-shaped electrode (201, 202, 203, 204, 205, 206, 207, 208, 209, 210,72, 73, 74) and thereby being coupled to said ring-shaped electrode (201, 202, 203, 204, 205, 206, 207, 208, 209, 210,72, 73, 74) while entering the lumen (12L) through the side hole (15) and extending in said lumen (12L), and
wherein the catheter shaft (10, 60) is constituted by a braided tube (13) that is reinforced by a braid (135, 65) made of a resin throughout the entire length thereof.

2. The electrode catheter (100) according to Claim 1, wherein the catheter shaft (10) has a guide-wire lumen (11L).

3. The electrode catheter (100) according to Claim 1, wherein the catheter shaft (10) comprises an inside tube (11) that has a guide-wire lumen (11L) and an outside tube (13) that forms, in cooperation with an outer circumferential surface of the inside tube (11), a lumen (12L) into which the lead (301, 302, 303, 304, 305, 306, 307, 308, 309, 310; 82, 83, 84) is inserted, the outside tube (13) being the braided tube (13).

4. The electrode catheter (100, 200) according to Claim 1, wherein the catheter shaft (60) is a multi-lumen tube that has a plurality of lumens (61L, 62L, 63L, 64L) inside the braid (65).

## Patentansprüche

1. Elektrodenkatheter (100, 200), umfassend:
einen Katheterschaft (10, 60), der mindestens ein Lumen (12L, 61L, 62L, 63L, 64L) aufweist; mindestens eine ringförmige Elektrode (201, 202, 203, 204, 205, 206, 207, 208, 209, 210; 72, 73, 74), die an einer äußeren Umfangsfläche eines distalen Endteils (101, 601) des Katheterschafts (10, 60) angebracht ist; und eine Leitung (301, 302, 303, 304, 305, 306, 307, 308, 309, 310; 82, 83, 84), die mit der ringförmigen Elektrode (201, 202, 203, 204, 205, 206, 207, 208, 209, 210; 72, 73, 74) gekoppelt ist,
wobei ein Seitenloch (15), das sich von der äußeren Umfangsfläche zu dem Lumen (12L) erstreckt, in einer Rohrwand des distalen Endteils (101, 601) des Katheterschafts (10, 60) in Übereinstimmung mit einer Befestigungsposition der ringförmigen Elektrode (201, 202, 203, 204, 205, 206, 207, 208, 209, 210; 72, 73, 74) ausgebildet ist,
wobei die Leitung (301, 302, 303, 304, 305, 306, 307, 308, 309, 310; 82, 83, 84) durch Beschichten (312) eines Metallkerndrahtes (311) mit einem Harz konfiguriert ist, wobei die Leitung (301, 302, 303, 304, 305, 306, 307, 308, 309, 310; 82, 83, 84) an einem distalen Endabschnitt davon mit einer inneren Umfangsfläche der ringförmigen Elektrode (201, 202, 203, 204, 205, 206, 207, 208, 209, 210; 72, 73, 74) verbunden ist und dadurch mit der ringförmigen Elektrode (201, 202, 203, 204, 205, 206, 207, 208, 209, 210; 72, 73, 74) gekoppelt ist, während sie durch das Seitenloch (15) in das Lumen (12L) eintritt und sich in dem Lumen (12L) erstreckt, und
wobei der Katheterschaft (10, 60) durch einen Gewebeschlauch (13) gebildet wird, der durch eine Umflechtung (135, 65) aus einem Harz über seine gesamte Länge verstärkt ist.

2. Elektrodenkatheter (100) nach Anspruch 1, wobei der Katheterschaft (10) ein Führungsdrahtlumen (11L) aufweist.

3. Elektrodenkatheter (100) nach Anspruch 1, wobei der Katheterschaft (10) einen Innenschlauch (11), der ein Führungsdrahtlumen (11L) aufweist, und einen Außenschlauch (13) umfasst, der im Zusammenwirken mit einer äußeren Umfangsfläche des Innenschlauchs (11) ein Lumen (12L) bildet, in das die Leitung (301, 302, 303, 304, 305, 306, 307, 308, 309, 310; 82, 83, 84) eingeführt wird, wobei der Außenschlauch (13) der Gewebeschlauch (13) ist.

4. Elektrodenkatheter (100, 200) nach Anspruch 1, wobei der Katheterschaft (60) ein mehrlumiger Schlauch ist, der innerhalb der Umflechtung (65) eine Vielzahl von Lumen (61L, 62L, 63L, 64L) aufweist.

## Revendications

1. Cathéter à électrode (100, 200) comprenant :
une tige de cathéter (10, 60) qui a au moins une lumière (12L, 61L, 62L, 63L, 64L) ; au moins une électrode en forme d'anneau (201, 202, 203, 204, 205, 206, 207, 208, 209, 210 ; 72, 73, 74) qui est fixée à une surface circonférentielle externe d'une partie d'extrémité distale (101, 601) de la tige de cathéter (10, 60) ; et un conducteur (301, 302, 303, 304, 305, 306, 307, 308, 309, 310 ; 82, 83, 84) qui est couplé à l'électrode en forme d'anneau (201, 202, 203, 204, 205, 206, 207, 208, 209, 210; 72, 73, 74),
dans laquelle un trou latéral (15) qui s'étend de la surface circonférentielle externe à la lumière (12L) est formé dans une paroi de tube de la partie d'extrémité distale (101, 601) de la tige de cathéter (10, 60) en correspondance avec une position de fixation de l'électrode en forme d'anneau (201, 202, 203, 204, 205, 206, 207, 208, 209, 210; 72, 73, 74),
dans laquelle le conducteur (301, 302, 303, 304, 305, 306, 307, 308, 309, 310 ; 82, 83, 84) est configuré par revêtement (312) d'un fil d'âme métallique (311) avec une résine, le conducteur (301, 302, 303, 304, 305, 306, 307, 308, 309, 310 ; 82, 83, 84) étant joint au niveau d'une partie d'extrémité distale de celui-ci à une surface circonférentielle interne de l'électrode en forme d'anneau (201, 202, 203, 204, 205, 206, 207, 208, 209, 210 ; 72, 73, 74) et étant de ce fait couplé à ladite électrode en forme d'anneau (201, 202, 203, 204, 205, 206, 207, 208, 209, 210 ; 72, 73, 74) en entrant dans la lumière (12L) par l'intermédiaire du trou latéral (15) et s'étendant dans ladite lumière (12L), et
dans laquelle la tige de cathéter (10, 60) est constituée d'un tube tressé (13) qui est renforcé par une tresse (135, 65) faite d'une résine sur l'entièreté de la longueur de celui-ci.

2. Cathéter à électrode (100) selon la revendication 1, dans laquelle la tige de cathéter (10) a une lumière de fil-guide (11L).

3. Cathéter à électrode (100) selon la revendication 1, dans laquelle la tige de cathéter (10) comprend un tube intérieur (11) qui a une lumière de fil-guide (11L) et un tube extérieur (13) qui forme, en coopération avec une surface circonférentielle externe du tube intérieur (11), une lumière (12L) dans laquelle le conducteur (301, 302, 303, 304, 305, 306, 307, 308, 309, 310 ; 82, 83, 84) est inséré, le tube extérieur (13) étant le tube tressé (13).

4. Cathéter à électrode (100, 200) selon la revendication 1, dans laquelle la tige de cathéter (60) est un tube à lumières multiples qui a une pluralité de lumières (61L, 62L, 63L, 64L) à l'intérieur de la tresse (65).
